# EUROPEAN PATENT APPLICATION

(11) **EP 2 044 895 A2**
(43) Date of publication of application: **08.04.2009**
(21) Application number: 08253186.4
(22) Date of filing: 30.09.2008
(51) Int. Cl.: A61B 17/34

(54) **Surgical portal kit for use in single incision surgery**

(30) Priority: 05.10.2007 US 998046 P; 20.08.2008 US 194831
(71) Applicant: Tyco Healthcare Group LP, North Haven, CT 06473 (US)
(72) Inventor: Richard, Paul D., Newtown CT 06470 (US); Stellon, Gene A., Burlington CT 06413 (US); Izzo, Steve L., Naugatuck CT 06770 (US)
(74) Representative: Soames, Candida Jane

(57) **Abstract**

A surgical kit (10) for performing a surgical procedure includes at least two portal members (100), possibly, at least three portal members, and an obturator (309) positionable within each of the portal members. Each portal member includes a portal housing (102) and a portal sleeve (104) extending from the portal housing, and having a passageway (110) therethrough for reception of a surgical object, the portal head having a reduced profile, an object seal (142) adapted to establish a fluid tight seal about the surgical object introduced therethrough and an insufflation port (126) for permitting passage of insufflation gases. At least one of the at least two portal members includes an insufflation plug (128). The insufflation plug (128) is positionable within the insufflation port (126) to substantially close the insufflation port.

## Description

### CROSS-REFERENCE TO RELATED APPLICATION

The present application claims the benefit of and priority to U.S. Provisional Application Serial No. 60/998,046, filed October 5, 2007, the entire content of which being incorporated herein by reference.

### BACKGROUND

### 1. Technical Field

The present disclosure relates to a surgical portal kit for use in a surgical procedure. More particularly, the present disclosure relates to a surgical portal kit including at least one access port with reduced dimensional features to enable positioning of multiple ports within a single incision.

### 2. Background of the Related Art

Today, many surgical procedures are performed through small incisions in the skin, as compared to the larger incisions typically required in traditional procedures, in an effort to reduce both trauma to the patient and recovery time. Generally, such procedures are referred to as "endoscopic", unless performed on the patient's abdomen, in which case the procedure is referred to as "laparoscopic". Throughout the present disclosure, the term "minimally invasive" should be understood to encompass both endoscopic and laparoscopic procedures.

During a typical minimally invasive procedure, surgical objects, such as surgical access devices, e.g. trocar and cannula assemblies, or endoscopes, are inserted into the patient's body through the incision in tissue. In laparoscopic surgery, several cannulas may be positioned at different abdominal locations to access the peritoneal cavity to permit the introduction of the surgical instruments. Unfortunately, the presence of multiple cannulas within the operative area limits maneuverability about the patient thereby potentially impeding the surgical procedure. Furthermore, the creation of multiple incisions to accommodate the cannulas may increase trauma to the patient and recovery time.

### SUMMARY

Accordingly, the present disclosure is directed to a surgical kit for performing a surgical procedure, e.g., a laparoscopic surgical procedure, through a single incision in the skin. The surgical kit includes at least two portal members, possibly, at least three portal members, and an obturator positionable within each of the portal members. Each portal member includes a portal housing and a portal sleeve extending from the portal housing, and having a passageway therethrough for reception of a surgical object, an object seal adapted to establish a fluid tight seal about the surgical object introduced therethrough and an insufflation port for permitting passage of insufflation gases. The portal head has a reduced profile. At least one of the at least two portal members includes an insufflation plug. The insufflation plug is positionable within the insufflation port to substantially close the insufflation port.

The surgical kit may include insufflation tubing. The insufflation tubing is adapted for mounting to the insufflation port of each of the at least two portal members and is connectable to a source of insufflation fluids. A clamp may be mountable on the insufflation tubing for selectively opening and closing a lumen of the insufflation tubing.

A zero closure valve may be mounted within each of the at least two portal members. The zero closure valve is adapted to close in the absence of a surgical object.

A method for perfoming a laparoscopic surgical procedure is disclosed. The method includes the steps of:
forming a single incision in skin tissue overlying the peritoneal cavity;
introducing at least two portal members through the single incision and advancing the at least two portal members through different openings of the peritonea fascia tissue to access the peritoneal cavity; and
performing surgical procedures through the at least two portal members.

The step of introducing may include introducing a third portal member through the single incision and advancing the third portal member through peritonea fascia tissue to access the peritoneal cavity.

### BRIEF DESCRIPTION OF THE DRAWINGS

Various embodiments of the present disclosure are described hereinbelow with references to the drawings, wherein:
**FIG.1** is a view of a surgical kit apparatus in accordance with the principles of the present disclosure illustrating at least two portal members, an insufflation tube and an obturator of the surgical kit;
**FIG. 2** is a side cross-sectional view of the housing of the portal member; and
**FIG. 3** is a flow chart illustrating use of the surgical kit in performing a laparoscopic surgical procedure; and
**FIG. 4** is a view illustrating positioning of multiple portal members within a single incision in the skin fascia in accordance with the method of using the surgical kit.

### DETAILED DESCRIPTION OF THE EMBODIMENTS

The surgical kit of the present disclosure is intended for use in the performance of a minimally invasive surgical procedure. The surgical kit permits the introduction and manipulation of various types of instrumentation while maintaining a fluid tight interface about the instrumentation to prevent gas and/or fluid leakage from the established pneumoperitoneum so as to preserve the atmospheric integrity of a surgical procedure. Examples of instrumentation include clip appliers, graspers, dissectors, retractors, staplers, laser probes, photographic devices, endoscopes and laparoscopes, tubes, and the like. Such instruments will be collectively referred to herein as "instruments or instrumentation".

In the drawings and in the description which follows, in which like reference numerals identify similar or identical elements, the term "proximal" or "trailing" will refer to the end of the apparatus which is closest to the clinician during use, while the term "distal" or "leading" will refer to the end which is furthest from the clinician.

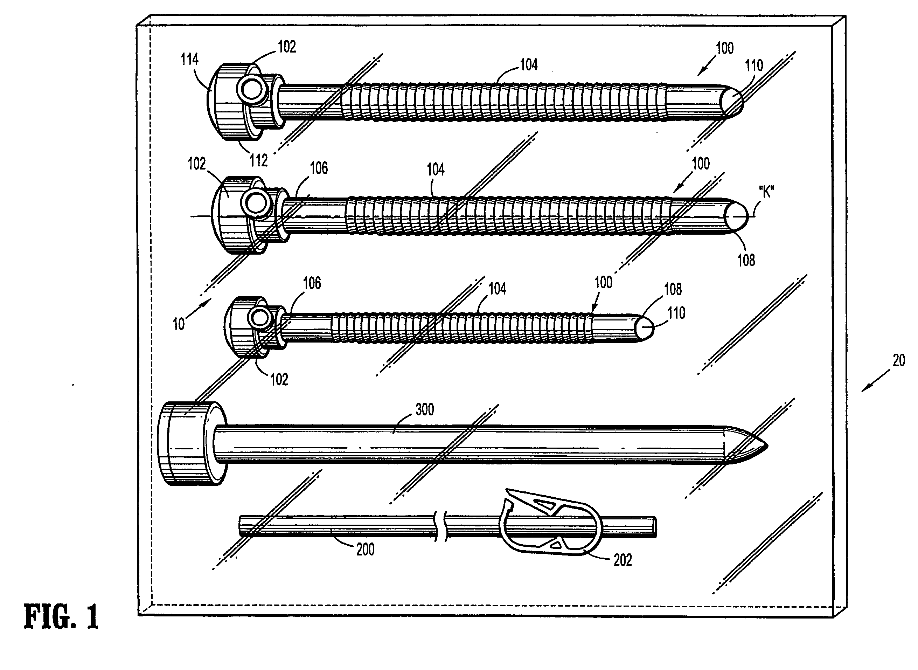

With reference to **FIG.1****,** a surgical kit **10** for use in a surgical procedure, e.g., a minimally invasive procedure is illustrated. Surgical kit **10** has particular application in laparoscopic surgery where the peritoneal cavity is insufflated to raise the cavity wall thereby providing access to tissue, organs etc within the cavity; however, uses in other minimally invasive procedures also are envisioned including arthroscopic, endoscopic or the like. Surgical kit **10** includes at least one, preferably, a plurality of portal members **100,** an insufflation tubing **200** for connection to at least one of the portal members **100** and an obturator **300.** The components of surgical kit **10** may vary in number or like depending on the surgical procedure to be performed. Various combinations of portal members **100,** insufflation tubing and obturators are envisioned. Surgical kit **10** may be packaged within a tray **20** or the like and may be provided as a unit for a specific surgical procedure. For example, the surgical kit 10 may include portal members 100 of various diameters and/or lengths that are suitable for specific surgical procedures, e.g., hernia, bariatric, etc. for specific individuals, e.g., children, adults, etc. or for any other criteria.

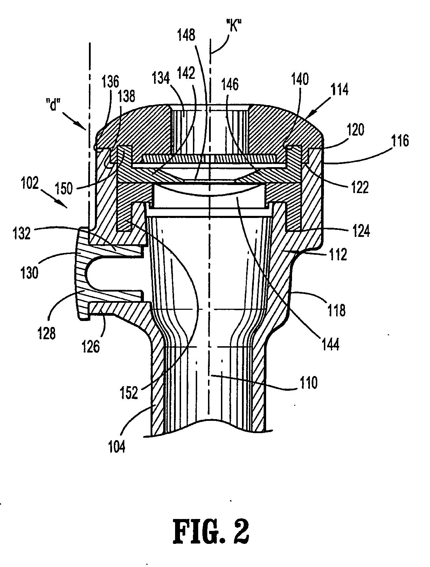

Referring now to **FIGS. 1-2****,** each portal member **100** includes portal head or housing **102** and portal sleeve **104** connected to the housing **102.** Portal sleeve **104** defines a longitudinal axis "k" extending along the length of the portal sleeve **104** and has proximal (or trailing) and distal (or leading) ends **106,108.** Portal sleeve **104** may be formed of any suitable medical grade material, such as stainless steel or other rigid materials, including polymeric materials, such as polycarbonate, or the like. Portal sleeve **104** may be transparent or opaque. The diameter of portal sleeve **104** may vary, but, typically ranges from about 3 millimeters (mm) to about **18** mm. In one embodiment, the diameter of portal sleeve **104** is about **5** mm.

Portal sleeve **104** may or may not include means for facilitating retention of the portal sleeve **104** within tissue. Such means may include a plurality of locking elements or ribs such as, e.g., the locking arrangement disclosed in commonly assigned U.S. Patent Application Serial No. 11/170,824 to Smith filed June 30, 2005**,** the entire contents of the **'824** disclosure being hereby incorporated by reference herein. Portal sleeve **104** and portal head **102** further define internal longitudinal passage **110** extending through the portal sleeve **104** and the portal head 102 dimensioned to permit passage of surgical instrumentation.

Portal head **102** includes portal base **112** and portal cap **114** which is releasably mounted to the portal base **112.** Any arrangement for mounting portal cap **114** to portal base **112** are envisioned including, but, not limited to, adhesives, cements, bayonet coupling, frictional fit, snap fit or the like. Portal head **102** defines first and second head segments **116, 118.** First head segment **116** defines a substantially circular cross-sectional dimension transverse to the longitudinal axis "k". In one embodiment, the maximum dimension or diameter of first head segment **116** ranges from about **5** millimeters (mm) to about **15** millimeters (mm), more preferably, about **8** millimeters (mm) to about 12 millimeters (mm). The maximum dimension or diameter of second head segment **118** ranges from about **3** millimeters (mm) to about **12** millimeters (mm), more preferably, about **5** millimeters (mm) to about **8** millimeters (mm). This dimensioning provides a substantially reduced profile to portal head **102** relative to conventional cannula assemblies thereby occupying substantially less space within the operative region above the operative site and facilitating the placement of multiple portal members **100** in adjacent sided by side relation within, e.g., a single incision, as will be discussed.

Portal base **112** defines outer peripheral shelf **120** extending orthogonal to longitudinal axis "k", a second step or shelf **122** inward of the outer annular shelf **120** and annular mounting recess **124** which is disposed inward of the second shelf **122.**

Portal base **112** further define insufflation port **126** which depends radially outwardly from second head segment **118.** Insufflation port **126** permits the introduction and/or release of insufflation gases through longitudinal passage **110** of portal member **100.** The disposition of insufflation port **126** adjacent second head segment **118** results in only a slight extension of the insufflation port **126** beyond the perimeter of first head segment **116.** In particular, insufflation port **126** extends a distance "d" beyond first head segment **116.** Distance "d" is substantially negligible ranging from about **1** millimeter (mm) to about **3** millimeter (mm) thereby also minimizing the profile of portal head **102** within the operative region and the potential of obstruction of the portal base **112** with activities, tasks performed during the surgical procedure. Insufflation port **126** may be supplied with insufflation plug **128** which is selectively positionable within the insufflation port **126.** Insufflation plug **128** may be fabricated from a suitable polymeric, elastomeric or foam material and is intended to close the insufflation port **126** to prevent leakage of insufflation gases. Insufflation plug **128** defines flat plug head **130** and plug extension **132** which is received within insufflation port **126.** Plug extension **132** is dimensioned to establish a sealing relation with the internal surface area of insufflation port **126.**

Portal cap **114** defines central opening **134** having an internal dimension or diameter approximating the internal diameter of portal sleeve **104.** The outer diameter of dimensioning of portal cap **114** generally approximates the outer diameter of portal base **112** as shown. Portal cap **114** defines outer peripheral shelf **136,** second shelf **138** disposed radially inward of the peripheral shelf **136** and annular mounting recess **140** which is inward of the second shelf **138.** Outer peripheral shelf **136** and second shelf **138** of portal cap **114** reside on respective outer peripheral shelf **120** and second shelf **122** of portal base **112** when in the assembled condition of the components. Portal cap **114** and portal base **112** may be adhered along respective shelves to secure the two components to each other.

Portal head **102** includes object seal **142** and zero closure valve **144.** Object seal **142** may be any seal adapted to form or establish a sealing relation with a surgical instrumentation introduced through portal member **100.** In one embodiment, object seal **142** is a septum seal defining inner seal segment **146** having central aperture **148.** Inner seal segment **146** defines a cross-sectional dimension or thickness which gradually decreases toward central aperture **148** and longitudinal axis "k". Object seal **142** may be fabricated from a suitable elastomeric material, gel material, foam material or a fluid filled cavity, having sufficient compliance to form a seal about the surgical instrumentation. Object seal **142** preferably comprises a resilient material in at least the region of inner seal segment **146** to form a substantial seal about an instrument inserted through central aperture **148.** Object seal **142** may be monolithically formed or composed of several components interconnected to each other. In one embodiment, object seal **142** includes a resilient elastomer (e.g., polyisoprene or natural rubber) and has a layer of fabric impregnated on each surface of the resilient seal. The fabric may be of any suitable fabric for example, a SPANDEX material containing about **20**% LYCRA and about **80**% NYLON available from Milliken. A suitable object seal is disclosed in commonly assigned U.S. Pat. No. 6,702,787 to Racenet et al. and/or U.S. Pat. No. 6,482,181 to Racenet et al., the entire contents of each disclosure being incorporated herein by reference.

Object seal **142** includes peripheral flange **150** extending in a proximal or trailing direction. Flange **150** is dimensioned to be received within annular mounting recess **140** of portal cap **114** to facilitate securement of object seal **142** within portal head **102.**

Zero closure valve **144** is mounted adjacent object seal **142** and may be in contacting relation with the object seal **142.** Zero closure valve **144** may be any valve adapted to close in the absence of the surgical object and/or in response to the pressurized environment of the underlying insufflated body cavity. Zero closure valve **144** may be a duck bill valve, trumpet valve, gel seal, foam seal, bladder seal or the like. In one embodiment, zero closure valve **144** includes outer peripheral flange **152** depending in a leading or distal direction. Flange **152** is received within corresponding annular **124** recess of portal base **112** to facilitate securement of the zero closure valve **144** within portal head **102.**

Portal head **102** is assembled by positioning zero closure valve **144** adjacent portal base **112** with peripheral flange **152** being received within annular mounting recess **124** of the portal base **112.** Zero closure valve **144** is placed in, e.g., superposed relation, with object seal **142.** Portal cap **114** is positioned on portal base **112** with peripheral flange **150** of object seal **142** being received within annular mounting recess **140** of portal cap **114.** Portal cap **114** is then secured relative to portal base **112** by any of the aforementioned means including, e.g., adhering the portal cap **114** and the portal base **112** along respective shelves.

Surgical kit **10** may incorporate portal members **100** identical in size and type, or alternatively, having different sizes, lengths, diameters etc.

Referring again to **FIG. 1****,** insufflation tubing **200** of surgical kit **10** is adapted for introduction within insufflation port **126** of portal base **112** and establishes a frictional fit with the interior wall of the insufflation port **126** to releasably secure the tubing **200** to portal head **102.** Other means for securing insufflation tubing **200** to portal head **102** are envisioned including bayonet coupling, snap fit or the like. Insufflation tubing **200** is connectable to a source of insufflation gases. Insufflation tubing **200** may further include tube clamp **202.** Tube clamp 202 is adapted to open and close to selectively open or close the lumen of insufflation tubing **200.** Any conventional tube or catheter clamp may be utilized.

Referring still to **FIG. 1****,** obturator **300** may be blunt, a non-bladed, or a sharp pointed instrument positionable within the passageway of the portal member **100.** Obturator **300** is utilized to penetrate the peritoneal wall to introduce portal member **100** through the peritoneal wall, and then subsequently is removed from the portal member **100** to permit introduction of the surgical instrumentation utilized to perform the procedure through the longitudinal passage **110** of the portal member **100.**

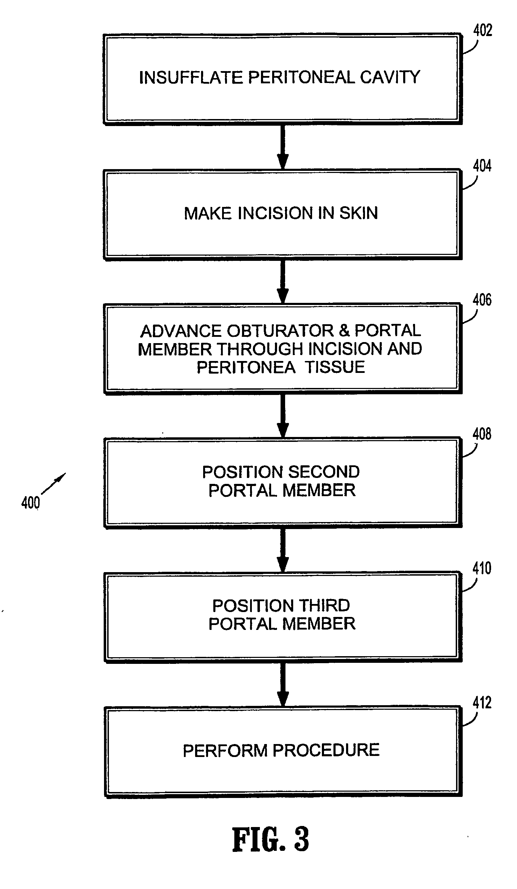

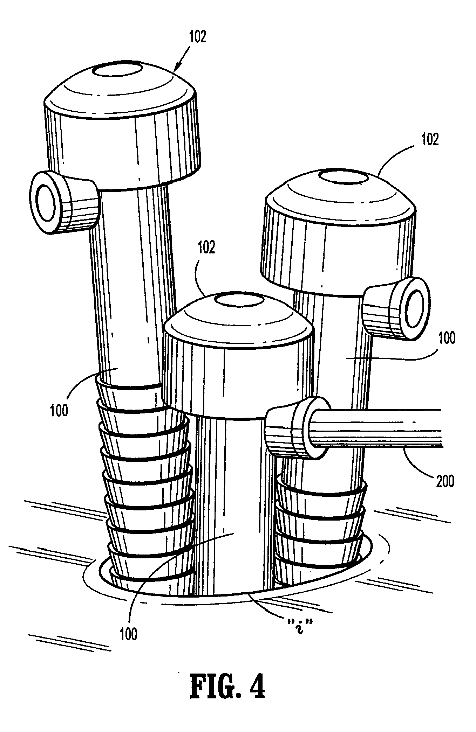

Referring now to the flow chart of **FIG. 3****,** the use and function of surgical kit **10** will be discussed during the course of a laparoscopic minimally invasive procedure (**STEP 400**). Initially, the peritoneal cavity is insufflated with a suitable biocompatible gas such as, e.g., CO₂ gas, such that the cavity wall is raised and lifted away from the internal organs and tissue to provide access the organs. (**STEP 402**) The insufflation may be performed with an insufflation needle or similar device, as is conventional in the art. Either prior or subsequent to insufflation, a single incision is made in at least the skin fascia, the dimensions of which may be varied dependent upon the nature of the procedure. (**STEP 404**) Thereafter, obturator 300 is mounted within portal member **100** and the assembled unit is positioned within the incision within the skin. Obturator **300** is manipulated through the skin incision to penetrate through deep fascia or peritonea tissue, e.g., the peritoneal lining, to access the underlying peritoneal cavity. (**STEP 406**) Thereafter, additional portal members **100** from the surgical kit **10** may be used with the obturator to access the peritoneal cavity in a similar manner. In one method of operation, a second portal member **100** is introduced within the previously formed skin incision and advanced in conjunction with the obturator to create another opening in the peritonea fascia to access the peritoneal cavity (**STEP 408**). This procedure thereby positions second portal member **100** in adjacent relation to first portal member **100.** A third portal member **100** optionally may be positioned within the same skin incision and advanced with the obturator to define another opening within the deep peritonea fascia tissue. (**STEP 410**) **FIG. 4** illustrates three portal members **100** accessing the peritoneal cavity. The respective low profile dimensioning of portal members **100,** particularly, portal heads **102** enables such placement of portal members **100** in side by side relation. Portal members **100** may be positioned at different depths with respect to the each other to laterally displace respective portal heads **102** of the portal members **100** to maximize available spacing within the operative region as depicted in **FIG. 4****.** One or more portal members **100** may have insufflation tubing **200** mounted to its insufflation port **126** to selectively supply insufflation gases to the peritoneal cavity. The remaining portal members **100** may have insufflation plugs **128** mounted within their respective insufflation ports **126** to prevent escape of insufflation gases through the insufflation ports **126.** Thereafter, surgery is performed with instrumentation positioned within any of portal members **100** accessing the peritoneal cavity. (**STEP 412**)

Although the illustrative embodiments of the present disclosure have been described herein with reference to the accompanying drawings, the above description, disclosure, and figures should not be construed as limiting, but merely as exemplifications of particular embodiments. It is to be understood, therefore, that the disclosure is not limited to those precise embodiments, and that various other changes and modifications may be effected therein by one skilled in the art without departing from the scope or spirit of the disclosure.

A surgical kit for performing a surgical procedure includes at least two portal members, possibly, at least three portal members, and an obturator positionable within each of the portal members. Each portal member includes a portal housing and a portal sleeve extending from the portal housing, and having a passageway therethrough for reception of a surgical object, the portal head having a reduced profile, an object seal adapted to establish a fluid tight seal about the surgical object introduced therethrough and an insufflation port for permitting passage of insufflation gases. At least one of the at least two portal members includes an insufflation plug. The insufflation plug is positionable within the insufflation port to substantially close the insufflation port.

## Claims

1. A surgical kit for performing a surgical procedure, which comprises:
at least two portal members, each portal member including:
a portal housing and a portal sleeve extending from the portal housing, and having a passageway therethrough for reception of a surgical object, the portal head having a reduced profile;
an object seal adapted to establish a fluid tight seal about the surgical object introduced therethrough; and
an insufflation port for permitting passage of insufflation gases; and
an obturator positionable within each of the at least two portal members.

2. The surgical kit according to claim **1** wherein at least one of the at least two portal members includes an insufflation plug, the insufflation plug positionable within the insufflation port to substantially close the insufflation port.

3. The surgical kit according to claim **2** including insufflation tubing, the insufflation tubing adapted for mounting to the insufflation port of each of the at least two portal members and connectable to a source of insufflation fluids.

4. The surgical kit according to claim **3** including a clamp mountable on the insufflation tubing for selectively opening and closing a lumen of the insufflation tubing.

5. The surgical kit according to claim **1** wherein including a zero closure valve mounted within each of the at least two portal members, the zero closure valve adapted to close in the absence of a surgical object.

6. The surgical kit according to claim **1** including at least three portal members.

7. The surgical kit according to claim **1** wherein the portal sleeves of the at least two portal members define different internal dimensions.

8. A method for perfoming a laparoscopic surgical procedure, comprising the steps of:
forming a single incision in skin tissue overlying the peritoneal cavity;
introducing at least two portal members through the single incision and advancing the at least two portal members through different openings of the peritonea fascia tissue to access the peritoneal cavity; and
performing surgical procedures through the at least two portal members.

9. The method according to claim **8** wherein the step of introducing includes introducing a third portal member through the single incision and advancing the third portal member through peritonea fascia tissue to access the peritoneal cavity.
